# EUROPEAN PATENT APPLICATION

(11) **EP 3 494 977 A1**
(43) Date of publication of application: **12.06.2019**
(21) Application number: 17837254.6
(22) Date of filing: 03.08.2017
(51) Int. Cl.: A61K 35/28, A61K 9/00

(54) **COMPOSITION FOR PREVENTING OR TREATING PULMONARY FIBROSIS INCLUDING EXOSOME EXTRACTED FROM ADIPOSE-DERIVED STEM CELL AS ACTIVE COMPONENT**

(30) Priority: 05.08.2016 KR 20160099778
(71) Applicant: Exostemtech Co., Ltd., Sangrok-gu Ansan-si, Gyeonggi-do 15588 (KR)
(72) Inventor: CHO, Yong Woo, Seongnam-si Gyeonggi-do 13530 (KR); CHOI, Ji Suk, Gunpo-si Gyeonggi-do 15803 (KR); YEOM, Seung Ho, Chuncheon-si Gangwon-do 15803 (KR); JUNG, Youn Jae, Gunpo-si Gyeonggi-do 15886 (KR)
(74) Representative: Diehl & Partner GbR
(86) International application number: PCT/KR2017/008383
(87) International publication number: WO 2018/026203

(57) **Abstract**

The present disclosure relates to a composition for preventing or treating pulmonary fibrosis, containing an exosome isolated from an adipose-derived stem cell as an active ingredient, a use of the exosome in preparation of a pharmaceutical for preventing or treating pulmonary fibrosis and a method for preventing pulmonary fibrosis, including a step of administering a pharmaceutical composition containing the exosome as an active ingredient to a subject. The exosome isolated from an adipose-derived stem cell according to the present disclosure contains growth factors related to the treatment of pulmonary fibrosis, such as HGF (hepatocyte growth factor) and IL-10, and can significantly reduce the fibrotic region in a pulmonary fibrosis-induced test animal model. Therefore, it can be usefully used in prevention or treatment of pulmonary fibrosis. In addition, in the treatment of pulmonary fibrosis using the exosome according to the present disclosure, there is little concern of side effects caused by steroid-based drugs and antioxidants used in typical treatment of pulmonary fibrosis and the exosome can be delivered naturally to the lungs through injection, inhalation, etc. Therefore, pulmonary fibrosis can be treated conveniently and economically.

## Description

### TECHNICAL FIELD

The present disclosure relates to a composition for preventing or treating pulmonary fibrosis, containing exosomes isolated from adipose-derived stem cells as an active ingredient, a use of the exosome in preparation of a pharmaceutical for preventing or treating pulmonary fibrosis and a method for preventing pulmonary fibrosis, including a step of administering a pharmaceutical composition containing the exosome as an active ingredient to a subject.

### BACKGROUND ART

Pulmonary fibrosis refers to a disease in which the lung tissue undergoes severe structural damage as fibrosis of the tissue is induced as chronic inflammatory cells infiltrate into the alveolar wall. As the fibrosis proceeds, the lung tissue becomes hard and the alveolar wall is thickened. As a result, oxygen supply in the blood is reduced, thereby leading to shortness of breath. At present, there is no therapeutic method for completely restoring the lung tissue which has undergone fibrosis. Except for some cases of early detection and treatment and lung transplantation, most of the patients die within 3-5 years after the onset of the symptoms.

For treatment of early detected pulmonary fibrosis, steroid-based drugs such as steroids, azathioprine or cyclophosphamide, antioxidants such as acetylcysteine, growth factors such as the cytokine IFN-γ, etc. are used. Although the treatment methods using the steroid-based drugs and the antioxidants have been consistently studied and reported since 2000, there is no drug with clearly proven efficacy. Currently available drugs are reported to cause systemic side effects when administered for a long period of time or cause tolerance side effects. The treatment method involving the administration of growth factors, which is drawing a lot of attentions recently, is a relatively fundamental therapeutic approach using the 'interferon' which suppresses the production of TGF-β known as an important factor in pulmonary fibrosis. Because this therapeutic approach is based on the analysis of the cause of the disease, it has fewer side effects than the therapeutic methods using the steroid-based drugs or the antioxidants and various treatment methods such as injection, aerosol, etc. have been reported. However, because the exact cause of pulmonary fibrosis is not known, the therapeutic effect of single ingredients such as the interferon may be temporary and may occur only in some patients. Therefore, consistent studies and clinical trials are required.

In order to overcome these limitations, researches for treating pulmonary fibrosis using stem cells capable of producing and regulating various factors and cultures of the stem cells were reported (Korean Patent Registration No. 10-0837167 and Korean Patent Publication No. 10-2009-0122415). However, the biggest problem of the stem cells is that their accurate efficacy cannot be confirmed because their survival rate in vivo is not uniform among patients and the risk of development of the stem cells to cancer cells cannot be avoided. In addition, the cultures containing various growth factors secreted from the stem cells may cause unexpected problems because they contain various cellular wastes in addition to the growth factors.

Under this background, the inventors of the present disclosure have made consistent efforts to develop a therapeutic agent for pulmonary fibrosis and, as a result, have identified that exosomes isolated from adipose-derived stem cells contain growth factors related to the treatment of pulmonary fibrosis, such as HGF (hepatocyte growth factor) and IL-10, and exhibits superior therapeutic effect for pulmonary fibrosis in a pulmonary fibrosis-induced test animal model.

### DISCLOSURE

### Technical Problem

The present disclosure is directed to providing a pharmaceutical composition for preventing or treating pulmonary fibrosis, which contains exosomes isolated from adipose-derived stem cells as an active ingredient.

The present disclosure is also directed to providing a use of the exosome in preparation of a pharmaceutical for preventing or treating pulmonary fibrosis.

The present disclosure is also directed to providing a method for preventing pulmonary fibrosis, which includes a step of administering a pharmaceutical composition containing the exosome as an active ingredient to a subject.

### Technical Solution

In order to solve the problems described above, the present disclosure provides a composition for preventing or treating pulmonary fibrosis, which contains exosomes isolated from adipose-derived stem cells as an active ingredient, a use of the exosome in preparation of a pharmaceutical for preventing or treating pulmonary fibrosis and a method for preventing pulmonary fibrosis, which includes a step of administering a pharmaceutical composition containing the exosome as an active ingredient to a subject.

Hereinafter, the present disclosure is described in detail.

In an aspect, the present disclosure provides a pharmaceutical composition for preventing or treating pulmonary fibrosis, which contains exosomes isolated from adipose-derived stem cells as an active ingredient.

In the present disclosure, the term "stem cell" refers to a cell that can not only self-renew but also differentiate into various cell types, including fat, bone marrow, cord blood, placenta, etc., due to its multipotency in the presence of an adequate signal under the environment of the cell and can be used to treat various cell-damaging diseases such as myocardial infarction, cerebral infarction, degenerative arthritis, bone fracture, etc.

In the present disclosure, the stem cell may be an autologous or allogeneic stem cell, and may be derived from any animal species including human and non-human mammals.

In the present disclosure, the term "adipose-derived stem cell" refers to a stem cell derived from an adipose tissue. The adipose tissue provides a good condition for harvesting stem cells because a large amount of tissue can be taken easily. In addition, the adipose-derived stem cell exhibits stable growth and proliferation when cultured and can differentiate into a variety of cell types. It is reported that the stem cell differentiates not only into adipose tissue but also into mesenchymal tissues such as cartilage, bone, nerve tissue, vascular tissue, etc. and other various tissues such as, liver, kidney, cardiovascular tissue, etc.

In the present disclosure, the term "exosome" refers to a 40-120 nm-sized nanovesicle secreted from cells and includes the immunologically important proteins main histocompatibility complex (MHC) and heat shock protein, anti-inflammatory microRNA which induces strong anti-tumor immune response and microRNA which regulates the accumulation of collagen. Also, it is known to be bound to other cells or tissues and play various roles such as transport of membrane components, proteins and RNAs, etc.

The exosome of the present disclosure is an exosome derived from a stem cell and may be derived from an adipose-derived stem cell, an umbilical cord-derived stem cell or a bone marrow-derived stem cell, although not being limited thereto.

Because the exosome derived from the stem cell contains genes, proteins, growth factors, etc. related with the proliferation, differentiation and regeneration of the corresponding stem cell, it can play an important role in tissue regeneration.

Specifically, an exosome containing genetic information, proteins and growth factors of an origin cell can be isolated from the adipose-derived stem cell of the present disclosure. The isolated exosome may have the basic characteristics of the stem cell and may contain growth factors, biologically active proteins, genes, etc., which are necessary for tissue regeneration.

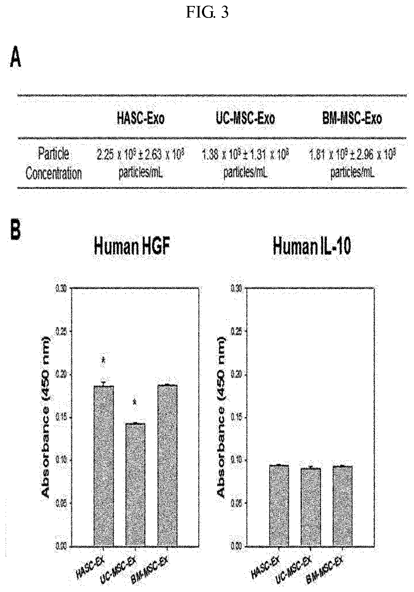

In an exemplary embodiment of the present disclosure, the exosome derived from an adipose-derived stem cell has been identified to contain growth factors related to the treatment of pulmonary fibrosis, such as HGF (hepatocyte growth factor), interleukin-10, etc. (FIG. 3, B).

The exosome may be obtained by an exosome isolation method known in the art. It may be obtained by an isolation method including the following steps, although not being limited thereto:
1) a step of culturing a stem cell in a normal culture medium and then subculturing in a serum-free, antibiotic-free medium;
2) a step of recovering the supernatant of the cell culture;
3) a step of removing cell debris by centrifuging the recovered supernatant of the cell culture; and
4) a step of obtaining an isolated/purified exosome by filtering the cell debris-removed cell culture supernatant through a multifilter system.

Specifically, in the step 1), the stem cell may be an adipose-derived stem cell, an umbilical cord-derived stem cell or a bone marrow-derived stem cell, although not being limited thereto. For example, it may be an adipose-derived stem cell.

The adipose-derived stem cell may be a stem cell derived from human or an animal.

In the step 1), the stem cell may be cultured under a normoxia condition or a hypoxic condition, although not being limited thereto.

The normoxia condition may be a culture condition of normal oxygen concentration, i.e., where 21% oxygen (O₂) is supplied, although not being limited thereto, or a culture condition wherein a hypoxic cell sensitizer is not added to a cell culture medium.

The hypoxic cell sensitizer is a material inducing a hypoxic condition and may be selected from a group consisting of cobalt chloride, desferrioxamine, and dimethyloxalylglycine (DMOG), although not being limited thereto.

The hypoxic condition may be a culture condition of low oxygen concentration, i.e., where 10% or less of oxygen (O₂) is supplied, although not being limited thereto, or a culture condition wherein a hypoxic cell sensitizer is added to a cell culture medium.

The low oxygen concentration may by oxygen concentration of 0.5-10%, 0.5-5% or 0.5-2%, although not being limited thereto.

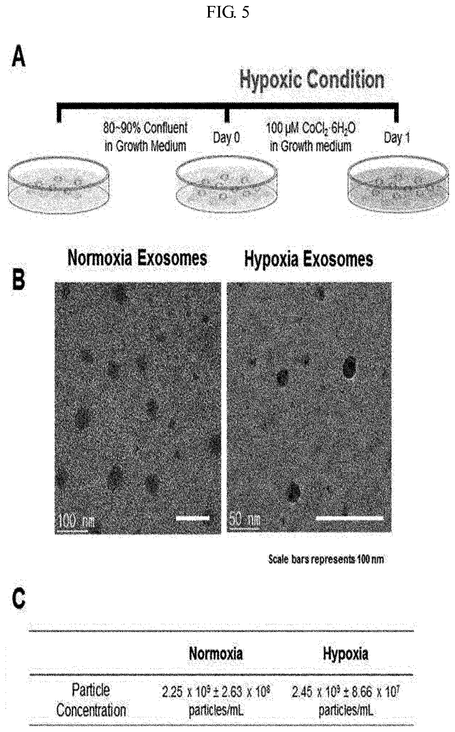

The hypoxic cell sensitizer is the same as described above. In an exemplary embodiment of the present disclosure, a hypoxic culture condition is induced by adding 100 µM cobalt chloride(II) hexahydrate (CoCl₂·6H₂O) to a medium for culturing an adipose-derived stem cell (FIG. 5, A).

In step 1), the normal culture medium may be any culture medium commonly used in the art. DMEM (Dulbecco's modified Eagle's medium), MEM (minimal essential medium) or RPMI 1640 (Roswell Park Memorial Institute 1640) may be used, although not being limited thereto.

If necessary, one or more adjuvant may be added to the cell culture medium. As the adjuvant, one or more selected from a group consisting of the serum of a bovine fetus, horse, human, etc., an antibiotic preventing bacterial contamination, such as penicillin G, streptomycin sulfate, gentamycin, etc., an antifungal agent such as amphotericin B, nystatin, etc. and a mixture thereof may be used.

Specifically, the culture medium may be DMEM (Dulbecco's modified Eagle's medium, high glucose) containing 10% FBS (fetal bovine serum) and 1% penicillin/streptomycin. In addition, it may be DMEM (Dulbecco's modified Eagle's medium, high glucose) not containing a serum, an antibiotic or phenol red, although not being limited thereto.

In an exemplary embodiment of the present disclosure, a human adipose-derived stem cell is cultured using DMEM (Dulbecco's modified Eagle's medium, high glucose) containing 10% FBS (fetal bovine serum) and 1% penicillin/streptomycin (passage 7 or 8) and then cultured for 24 hours in a serum-free, antibiotic-free, phenol red-free medium before isolating an exosome from the stem cell (Example 1).

The cell culture supernatant recovered in the step 2) contains the cell debris and the exosome secreted from the stem cell and the cell debris contained in the cell culture supernatant may be removed by centrifugation in the step 3).

The exosome contained in the cell culture supernatant recovered in the step 2) may be finally isolated and purified through centrifugation and filtration through a multifilter system.

The centrifugation may be performed at 250-500 g for 5-10 minutes, or at 300 g for 10 minutes, although not being limited thereto.

The multifilter system is a system of filtering through filters of different pore sizes step by step. For example, it may be a system of filtering through a cell strainer with a pore size of 4 µm, a filter with a pore size of 0.22 µm and a filter with a MWCO (molecular weight cut off) of 500 kD step by step, although not being limited thereto.

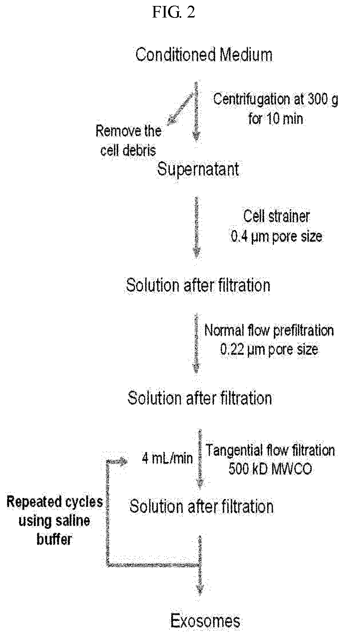

In an exemplary embodiment of the present disclosure, the cell culture supernatant recovered from the cultured human adipose-derived stem cell is centrifuged at 300 g for 10 minutes to remove cell debris. Then, a cell strainer with a pore size of 4 µm is used to remove the cell debris larger than the pore size and then a filter with a pore size of 0.22 µm is used to remove the cell debris larger than the pore size. The solution recovered after removing the cell debris is subjected to TFF (tangential flow filtration) through a filter with a MWCO (molecular weight cut off) of 500 kD at a flow rate of 4 mL/min to remove proteins. The solution recovered after the TFF process is sonicated. This TFF process is repeated continuously to obtain the finally isolated and purified exosome (FIG. 2).

The exosome of the present disclosure obtained through the exosome isolation method described above may be an exosome isolated from an adipose-derived stem cell cultured under a normoxia condition or a hypoxic condition.

The exosome may contain growth factors related to the treatment of pulmonary fibrosis, such as HGF (hepatocyte growth factor), interleukin-10, etc. Therefore, the exosome may be used as an active ingredient in a composition for preventing or treating pulmonary fibrosis.

In the present disclosure, the term "pulmonary fibrosis" refers to a disease characterized by diffuse fibroplasia in the alveolar wall, with the symptoms of dry cough or shortness of breath during working. It refers to the end of interstitial pneumonia in a narrow sense, but in a wide sense, it means the concomitant presence of the pulmonary fibrosis in a narrow sense and interstitial pneumonia. Any interstitial pneumonia can cause this pulmonary fibrosis. The interstitial pneumonia collectively refers to diseases causing inflammation in the interstitial of the lungs (including alveolar wall in a narrow sense and pleura, etc. in a wide sense) and includes interstitial pneumonia with a particular cause such as infection, diffuse collagen disease, radiation, medication, dust etc. and idiopathic interstitial pneumonia with an unknown cause. The idiopathic interstitial pneumonia is classified into idiopathic pulmonary fibrosis (IPF), nonspecific interstitial pneumonia (NSIP), acute interstitial pneumonia (AIP), cryptogenic organizing pneumonia (COP), respiratory bronchiolitis-associated interstitial lung disease (RB-ILD), desquamative interstitial pneumonia (DIP), lymphoid interstitial pneumonia (LIP), etc. based on video-assisted thoracoscopic surgery (VATS), high-resolution computed tomography (HRCT), etc.

The pharmaceutical composition for preventing or treating pulmonary fibrosis according to the present disclosure is distinguished from the existing technologies in that an exosome isolated from an adipose-derived stem cell cultured under a specific culture condition such as a normoxia condition or a hypoxic condition is used as an active ingredient for effective treatment of pulmonary fibrosis.

The isolated and purified exosome contains various genetic information, proteins and growth factors for the alleviation or treatment of pulmonary fibrosis. In addition, it exhibits excellent biocompatibility and superior absorption rate because it is a cell-derived substance.

Accordingly, the problems that may be caused by the currently available therapeutic agents for treatment of pulmonary fibrosis or the development of stem cells to cancer cells that may occur during treatment using the currently studied stem cell cultures or the side effects by cellular waste, etc. may be minimized.

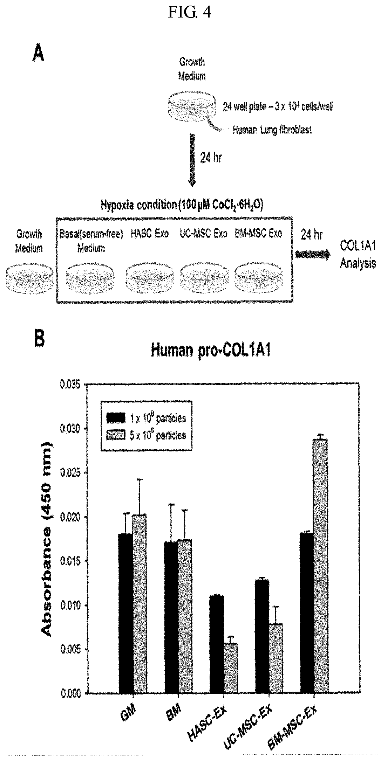

In an exemplary embodiment of the present disclosure, the exosome isolated from an adipose-derived stem cell has been confirmed to contain growth factors related to the treatment of pulmonary fibrosis, such as HGF (hepatocyte growth factor), interleukin-10, etc. (FIG. 3, B) and it has been confirmed that, in fibrosis-induced lung fibroblasts, the exosome isolated from an adipose-derived stem cell inhibits the synthesis of collagen, which is accumulated during fibrosis, thereby destroying the normal structure and bringing functional disorder (FIG. 4, B).

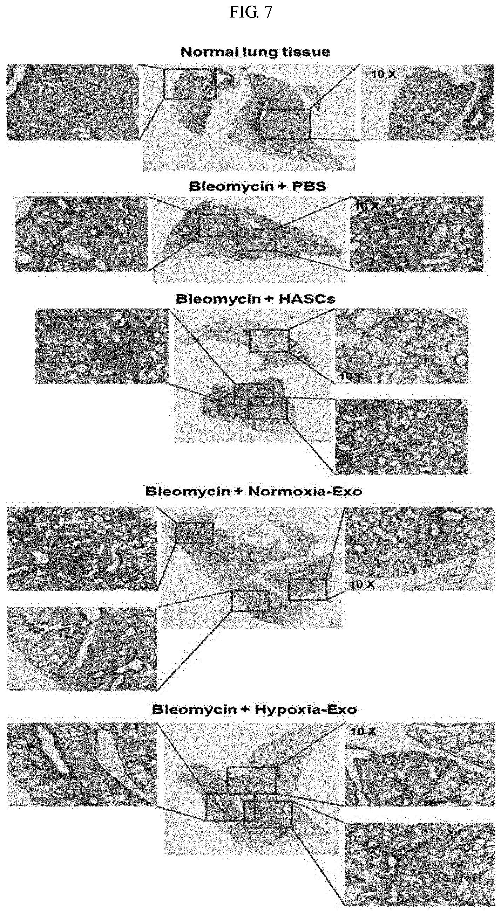

In addition, when Normoxia-Exo and Hypoxia-Exo respectively isolated from an adipose-derived stem cell under a normoxia condition and a hypoxic condition were administered to a bleomycin-induced pulmonary fibrosis C57BL/6J mouse and then trichrome staining was conducted for the lung tissue, the stained collagen area was reduced significantly similar to that of the normal lung tissue as compared to a negative control group (PBS-administered group) and a positive control group (adipose-derived stem cell-administered group). In particular, when Hypoxia-Exo was administered, the stained collagen area was reduced significantly similar to that of the normal lung tissue as compared to the negative control group, the positive control group and the Normoxia-Exo-administered group (FIGS. 7-9).

Based on the above results, it can be seen that both the exosome obtained from the adipose-derived stem cell cultured under a normoxia condition (Normoxia-Exo) and the exosome obtained from the adipose-derived stem cell cultured under a hypoxic condition (Hypoxia-Exo) are effective in improving or treating pulmonary fibrosis. In particular, it can be seen that the adipose-derived stem cell cultured under a hypoxic condition (Hypoxia-Exo) exhibits remarkably superior therapeutic effect for pulmonary fibrosis.

Accordingly, a composition containing the exosome isolated from an adipose-derived stem cell according to the present disclosure as an active ingredient may be usefully used as a pharmaceutical composition for preventing or treating pulmonary fibrosis.

The pharmaceutical composition for preventing or treating pulmonary fibrosis according to the present disclosure may contain a pharmaceutically effective amount of the exosome isolated from an adipose-derived stem cell either alone or together with one or more pharmaceutically acceptable carrier, excipient or diluent. Here, the pharmaceutically effective amount means an amount sufficient for preventing, improving or treating the symptoms of pulmonary fibrosis.

The pharmaceutically effective amount of the exosome isolated from an adipose-derived stem cell according to the present disclosure may be 5×10⁸-5×10¹⁰ particles/day/kg body weight or 5×10⁹ particles/day/kg body weight. However, the pharmaceutically effective amount may be changed adequately depending on the severity of the symptoms of pulmonary fibrosis, the age, body weight, health condition and sex of a patient, administration route, treatment period, etc.

And, the term "pharmaceutically acceptable" means that the composition is physiologically acceptable and, when administered to human, normally does not cause allergic reactions such as gastroenteric trouble or dizziness or similar reactions. Examples of the carrier, excipient and diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate and mineral oil. In addition, a filler, an antiflocculant, a lubricant, a humectant, a flavorant, an emulsifier, an antiseptic, etc. may be further included.

In addition, the composition of the present disclosure may be prepared into a unit-dose formulation suitable to be administered into the body of a patient according to a method commonly employed in the pharmaceutical field and the formulation contains an administration amount effective for developing alveoli through one or several administration(s). Specifically, formulations suitable for this purpose may be parenteral formulations such as an injection like an injection ampoule, an infusion such as an infusion bag, a spray such as an aerosol, etc. The injection ampoule may be mixed with an injection solution immediately prior to use and physiological saline, glucose, mannitol, Ringer's solution, etc. may be used as the injection. The infusion bag may be made of polyvinyl chloride or polyethylene. For example, an infusion bag available from Baxter, Becton-Dickinson, Medcep, National Hospital Products or Terumo may be used.

The pharmaceutical formulation may further contain, in addition to the active ingredient, one or more pharmaceutically acceptable common inert carrier, e.g., a preservative, a pain reliever, a solubilizer, a stabilizer, etc. for an injection or a base, an excipient, a lubricant or a preservative, etc. for a topical formulation.

The composition or pharmaceutical formulation of the present disclosure prepared as described above may be administered to a mammal such as rat, mouse, livestock, human, etc. through various routes including parenteral and oral routes. Any mode of administration commonly employed in the art may be used. For example, the administration may be made orally, rectally, intravenously, intramuscularly, subcutaneously, intrauterinely, intracerebroventricularly, etc., although not being limited thereto.

Specifically, the exosome isolated from an adipose-derived stem cell may be administered intravenously (intravenous injection), administered into the lungs or trachea of a subject (topical administration) or administered through inhalation. Also, the exosome may be administered using a nebulizer or may be administered using an endotracheal tube.

In another aspect, the present disclosure provides an injection for preventing or treating pulmonary fibrosis, which contains an exosome isolated from an adipose-derived stem cell as an active ingredient.

The injection may further contain phosphate-buffered saline (PBS). That is to say, the injection may be prepared by adding the exosome isolated from an adipose-derived stem cell to phosphate-buffered saline.

The injection may contain a hydrogel instead of the phosphate-buffered saline.

The hydrogel may be one or more selected from a group consisting of hyaluronic acid, gelatin, alginate, chitosan, fibrin, elastin, collagen and methyl cellulose, specifically hyaluronic acid, although not being limited thereto.

The injection may contain the exosome at a concentration of 5×10⁸-5×10¹⁰ particles/kg or 5×10⁹ particles/kg, although not being limited thereto.

The injection may be administered to the damaged part, e.g., lung, of a mammal such as rat, mouse, livestock, human, etc. or may be administered intravenously.

In another aspect, the present disclosure provides a use of an exosome isolated from an adipose-derived stem cell in preparation of a pharmaceutical for preventing or treating pulmonary fibrosis.

The exosome isolated from an adipose-derived stem cell is the same as described above and it may be used as an active ingredient of a pharmaceutical composition for preventing or treating pulmonary fibrosis as described above.

In another aspect, the present disclosure provides a method for treating pulmonary fibrosis using a pharmaceutical composition containing an exosome isolated from an adipose-derived stem cell as an active ingredient.

The method includes a step of administering the pharmaceutical composition to a subject according to various methods as described above. The specific administration dosage may vary depending on the condition and body weight of a subject, the severity of a disease, pharmaceutical type, administration route and administration period. The administration dosage may be selected adequately by those skilled in the art and the subject refers to any animal including human in which pulmonary fibrosis has occurred or may occur.

Specifically, the pharmaceutical composition may be administered to the subject through intravenous injection, inhalation, topical administration, etc. Because the exosome can be delivered directly to the lungs through inhalation or topical administration or, when injected intravenously, is accumulated naturally in the lungs while circulating through the blood vessels, pulmonary fibrosis can be treated effectively by a simple method as described above without a complicated treatment procedure.

Unless otherwise defined, all technical and scientific terms used in the present disclosure have the same meaning as commonly understood by those skilled in the art to which the present disclosure belongs.

### Advantageous Effects

An exosome isolated from an adipose-derived stem cell according to the present disclosure contains growth factors related to the treatment of pulmonary fibrosis, such as HGF (hepatocyte growth factor) and IL-10, and can significantly reduce the fibrotic region in a pulmonary fibrosis-induced test animal model. Therefore, it can be usefully used in prevention or treatment of pulmonary fibrosis. In addition, in the treatment of pulmonary fibrosis using the exosome according to the present disclosure, there is little concern of side effects caused by steroid-based drugs and antioxidants used in typical treatment of pulmonary fibrosis and the exosome can be delivered naturally to the lungs through injection, inhalation, etc. Therefore, pulmonary fibrosis can be treated conveniently and economically.

### DESCRIPTION OF DRAWINGS

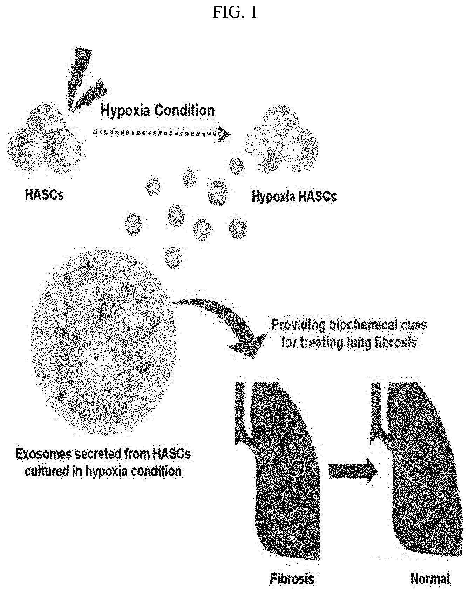

FIG. 1 schematically illustrates an exosome isolated from human adipose-derived stem cells (HASCs) and application thereof according to an exemplary embodiment of the present disclosure.

FIG. 2 schematically illustrates a method for isolating an exosome from stem cells of various origins according to an exemplary embodiment of the present disclosure.

FIG. 3 shows a result of analyzing the characteristics of exosomes isolated from a human adipose-derived stem cell (HASC), a human umbilical cord-derived mesenchymal stem cell (UC-MSC) and a human bone marrow-derived mesenchymal stem cell (BM-MSC) according to an exemplary embodiment of the present disclosure: (A) concentration of exosomes isolated from the stem cells of various origins determined by nanoparticle tracking analysis; (B) expression level of HGF (hepatocyte growth factor) and IL-10 (interleukin-10) exosomes determined by ELISA analysis.

FIG. 4 shows a result of analyzing collagen synthesis inhibition ability of exosomes isolated from a human adipose-derived stem cell (HASC), a human umbilical cord-derived mesenchymal stem cell (UC-MSC) and a human bone marrow-derived mesenchymal stem cell (BM-MSC) using a fibrosis-induced lung fibroblast according to an exemplary embodiment of the present disclosure: (A) schematic of hypoxic condition of lung fibroblast for inducing fibrosis and exosome treatment condition; (B) expression level of COL1A1 (human procollagen type I alpha 1) obtained by treating fibrosis-induced lung fibroblast with exosomes isolated from HASC, UC-MSC and BM-MSC and subjecting culture medium obtained 24 hours later to ELISA analysis. Here, GM represents a positive control group for which a growth medium was used and BM represents a negative control group for which a serum-free basal medium was used.

FIG. 5 shows a result of analyzing the characteristics of normoxia exosomes and hypoxia exosomes isolated from human adipose-derived stem cells cultured under normoxia and hypoxia conditions, respectively, according to an exemplary embodiment of the present disclosure: (A) schematic of culture condition and condition for isolation of exosomes from human adipose-derived stem cells (DMEM (high glucose) normal culture medium containing 10% FBS and 1% antibiotics is used for isolation of normoxia exosomes and normal culture medium supplemented with 100 µM cobalt chloride(II) hexahydrate (CoCl₂·6H₂O) is used for isolation of hypoxia exosomes. After culturing the human adipose-derived stem cells for 24 hours, the medium is replaced with a serum-free medium and the exosomes are isolated.); (B) transmission electron microscopic images showing the structure and morphology of normoxia exosomes and hypoxia exosomes; (C) concentration of normoxia exosomes and hypoxia exosomes determined by nanoparticle tracking analysis.

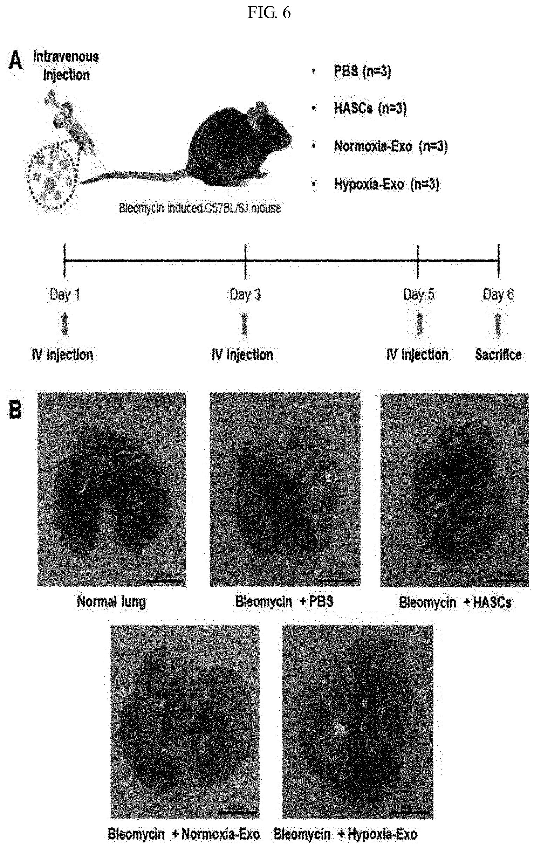

FIG. 6 shows the therapeutic effect for pulmonary fibrosis of an exosome isolated from an adipose-derived stem cell according to an exemplary embodiment of the present disclosure: (A) schematic of establishment of bleomycin-induced pulmonary fibrosis C57BL/6J mouse model and administration schedule of normoxia exosomes and hypoxia exosomes isolated from human adipose-derived stem cells; (B) images of mouse lungs obtained by administering normoxia exosomes and hypoxia exosomes isolated from human adipose-derived stem cells to bleomycin-induced pulmonary fibrosis C57BL/6J mouse for 3 times via intravenous injection with 2-day intervals and imaging the lungs of the mouse 1 day later (PBS represents a negative control group administered with PBS (phosphate buffer saline) (N = 3) and HASCs represents a positive control group administered with human adipose-derived stem cells at a concentration of 1×10⁶ cells/100 µL (N = 3). Normoxia-Exo and Hypoxia-Exo respectively represent test groups administered with normoxia exosomes and hypoxia exosomes at a concentration of 1×10⁸ particles/100 µL (N = 3). The PBS, human adipose-derived stem cells (HASCs), normoxia exosomes and hypoxia exosomes were administered to the bleomycin-induced pulmonary fibrosis C57BL/6J mouse for 3 times via intravenous injection with 2-day intervals.).

FIG. 7 shows the therapeutic effect for pulmonary fibrosis of an exosome isolated from an adipose-derived stem cell according to an exemplary embodiment of the present disclosure. After administering normoxia exosomes and hypoxia exosomes isolated from human adipose-derived stem cells to bleomycin-induced pulmonary fibrosis C57BL/6J mouse for 3 times via intravenous injection with 2-day intervals, trichrome staining was conducted on the lungs of the mouse 1 day later (magnification: ×10) (PBS represents a negative control group administered with PBS (phosphate buffer saline) (N = 3) and HASCs represents a positive control group administered with human adipose-derived stem cells at a concentration of 1×10⁶ cells/100 µL (N = 3). Normoxia-Exo and Hypoxia-Exo respectively represent test groups administered with normoxia exosomes and hypoxia exosomes at a concentration of 1×10⁸ particles/100 µL (N = 3).

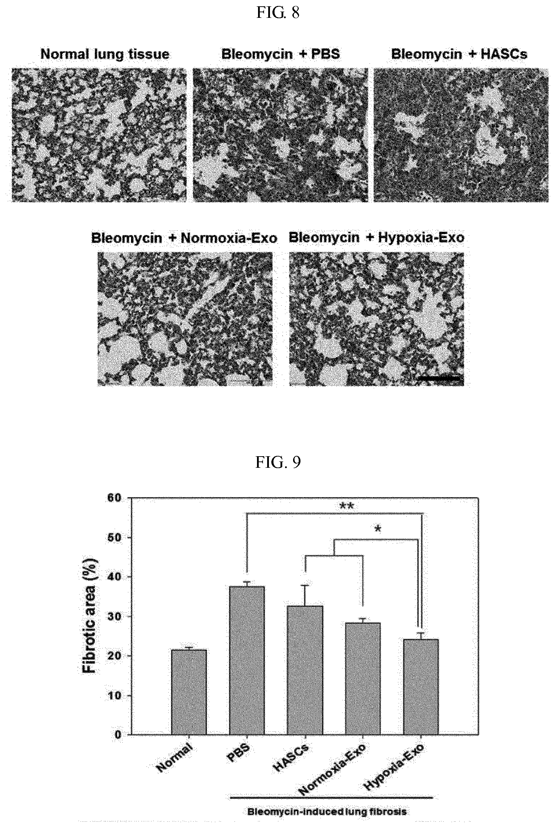

FIG. 8 shows the therapeutic effect for pulmonary fibrosis of an exosome isolated from an adipose-derived stem cell according to an exemplary embodiment of the present disclosure. After administering normoxia exosomes and hypoxia exosomes from human adipose-derived stem cells to bleomycin-induced pulmonary fibrosis C57BL/6J mouse for 3 times via intravenous injection with 2-day intervals, trichrome staining was conducted on the lungs of the mouse 1 day later (magnification: ×40; scale bar: 100 µm).

FIG. 9 shows the therapeutic effect for pulmonary fibrosis of an exosome isolated from an adipose-derived stem cell according to an exemplary embodiment of the present disclosure. After administering normoxia exosomes and hypoxia exosomes isolated from human adipose-derived stem cells to bleomycin-induced pulmonary fibrosis C57BL/6J mouse for 3 times via intravenous injection with 2-day intervals, trichrome staining was conducted on the lungs of the mouse 1 day later and the stained collagen area, i.e., the fibrotic area (%), was quantified (PBS represents a negative control group administered with PBS (phosphate buffer saline) (N = 3) and HASCs represents a positive control group administered with human adipose-derived stem cells at a concentration of 1×10⁶ cells/100 µL (N = 3). Normoxia-Exo and Hypoxia-Exo respectively represent test groups administered with normoxia exosomes and hypoxia exosomes at a concentration of 1×10⁸ particles/100 µL (N = 3).).

### BEST MODE

Hereinafter, the constitution and effect of the present disclosure will be described in more detail through examples. The examples are for illustrative purposes only and the scope of the present disclosure is not limited by the examples.

### Example 1: Isolation of exosomes from stem cells of various origins

Stem cells exhibit different characteristics depending on the tissue from which they are derived and the culturing condition. Therefore, the exosomes isolated from the stem cell exhibiting different characteristics show difference in structural components (Extracellular vesicles: Exosomes, microvesicles, and friends; G Raposo et al., J. Cell Biol., 2013, Vol. 200, No. 4, pp. 373-383). That is to say, the cell type that is the origin of the exosome is a very important factor determining the characteristics of the exosome.

Therefore, exosomes were derived from stem cells of various origins in order to isolate exosomes exhibiting effective therapeutic effect for pulmonary fibrosis.

Specifically, while culturing human adipose-derived stem cells (HASCs), human umbilical cord-derived mesenchymal stem cells (UC-MSCs) and human bone marrow-derived mesenchymal stem cells (BM-MSCs), exosomes were isolated from the respective stem cells.

The respective stem cells were cultured using a DMEM (Dulbecco's modified Eagle's medium, high glucose; Gibco, Cat#: 11995065) normal culture medium supplemented with 10% FBS (fetal bovine serum) and 1% penicillin/streptomycin.

24 hours before isolating exosomes from the respective stem cells, the medium was replaced with a serum-free, antibiotic-free, phenol red-free medium (Gibco, Cat#: 31053028). After culturing for 24 hours, a cell culture supernatant was recovered. The recovered cell culture supernatant was centrifuged at 300 g for 10 minutes to remove cell debris and then a cell strainer with a pore size of 0.4 µm was used to remove the debris larger than the pore size. Then, a filter with a pore size of 0.22 µm was used to remove the cell debris larger than the pore size. After the filtration, the recovered solution was subjected to TFF (tangential flow filtration) at a flow rate of 4 mL/min using a filter with a MWCO (molecular weight cut off) of 500 kD to remove proteins. The recovered solution was sonicated. The TFF process was repeated continuously to obtain the finally isolated and purified exosomes (FIG. 2).

After recovering the supernatant, the respective stem cells were cultured again after adding a normal culture medium. Subculturing was conducted until passage 7 or 8.

### Example 2: Characterization of exosomes isolated from stem cells of various origins

The characteristics of the exosomes isolated from the stem cells of various origins in Example 1 were analyzed.

First, the concentration of the exosomes isolated from the human adipose-derived stem cells (HASC-Exo), the exosomes isolated from the human umbilical cord-derived stem cells (UC-MSC-Exo) and the exosomes isolated from the human bone marrow-derived stem cells (BM-MSC-Exo) was investigated by nanoparticle tracking analysis.

As a result of investigating the concentration of the respective exosomes by nanoparticle tracking analysis, the concentration of the human adipose-derived stem cell exosomes (HASC-Exo) was 2.25×10⁹ particles/mL, the concentration of the human umbilical cord-derived stem cell exosomes (UC-MSC-Exo) was 1.38×10⁹ particles/mL and the concentration of the human bone marrow-derived stem cell exosomes (BM-MSC-Exo) was 1.81×10⁹ particles/mL (FIG. 3, A).

In addition, ELISA (enzyme-linked immunosorbent assay) was conducted in order to compare the characteristics of the exosomes isolated from the human adipose-derived stem cells, the human umbilical cord-derived stem cells and the human bone marrow-derived stem cells. Through this, the expression level of HGF (hepatocyte growth factor) and IL-10 (interleukin-10) known to be helpful in relieving pulmonary fibrosis was determined.

Specifically, after mixing the respective exosomes isolated from the stem cells of various origins at a concentration of 7×10⁷ particles/200 µL with a RIPA (radioimmunoprecipitation assay) buffer at a ratio of 1:1, the expression level of HGF and IL-10 in the exosomes was investigated using a HGF ELISA kit (Abcam, ab100534) and an IL-10 ELISA kit (Abcam, ab100549).

As a result of investigating the expression level of HGF and IL-10 by ELISA, HGF was expressed at the highest level in the exosomes isolated from the human adipose-derived stem cells (HASC-Exo), showing significant difference from the exosomes isolated from the human umbilical cord-derived stem cells (UC-MSC-Exo). Meanwhile, the expression level was not significantly different from the exosomes isolated from the human bone marrow-derived stem cells (BM-MSC-Exo). As for IL-10, the expression level was similar in all the exosomes isolated from the stem cells of various origins (FIG. 3, B).

### Example 3: Evaluation of collagen synthesis inhibition ability of exosomes using fibrosis-induced lung fibroblasts

Most organs undergo inflammation and healing processes after tissue damage. If the damage is slight, the normal structure and function of the tissue are maintained. But, continued damage leads to fibrosis of the tissue during the healing process. The fibrosis process involves the accumulation of extracellular matrices (ECM) such as collagen, fibronectin, etc. in the tissue, leading to destruction of normal structure and functional disorder (Tuberculosis and Respiratory Diseases, Vol. 54, No. 2, pp. 1-12, Feb, 2003).

In order to investigate whether the respective exosomes isolated from the human adipose-derived stem cells, the human umbilical cord-derived stem cells and the human bone marrow-derived stem cells exhibit effective therapeutic effect for pulmonary fibrosis, an *in-vitro* model was established using lung fibroblasts and the collagen synthesis inhibition ability of the respective exosomes isolated from the stem cells of various origins was evaluated.

Specifically, primary human lung fibroblasts (Normal, ATCC, PCS-201-013) were subcultured until passage 6 using a fibroblast basal medium (ATCC, PCS-201-030) supplemented with the fibroblast growth kit-low serum (ATCC, PCS-201-041).

After the subculturing, the medium composition was changed by adding cobalt chloride(II) hexahydrate ((CoCl₂·6H₂O) known as a lung sensitizer inducing a hypoxic condition in cultured cells to the culture medium at a concentration of 100 µM. The medium was treated with the exosomes isolated from the human adipose-derived stem cells (HASC-Exo), the exosomes isolated from the human umbilical cord-derived stem cells (UC-MSC-Exo) or the exosomes isolated from the human bone marrow-derived stem cells (BM-MSC-Exo) at a concentration of 1×10⁸ particles/mL or 5×10⁸ particles/mL. 24 hours later, the culture medium was harvested and the expression level of COL1A1 (collagen type I, alpha 1) was investigated using an ELISA kit. A growth medium (GM) and a serum-free basal medium (BM) were used as a positive control group and a negative control group, respectively.

As a result of evaluating the collagen synthesis inhibition ability of the exosomes isolated from the stem cells of various origins by ELISA, the group treated with the exosomes isolated from the human adipose-derived stem cells (HASC-Exo) showed significantly decreased expression of COL IA1 than the groups treated with the exosomes isolated from the human umbilical cord-derived stem cells (UC-MSC-Exo) and the exosomes isolated from the human bone marrow-derived stem cells (BM-MSC-Exo).

From this result, it was confirmed that the exosomes isolated from the human adipose-derived stem cells (HASC-Exo) exhibit superior collagen synthesis inhibition ability as compared to the exosomes isolated from the human umbilical cord-derived stem cells (UC-MSC-Exo) and the exosomes isolated from the human bone marrow-derived stem cells (BM-MSC-Exo) (FIG. 4).

Because the exosomes isolated from the human adipose-derived stem cells (HASC-Exo) exhibit superior collagen synthesis inhibition ability in the fibrosis-induced lung fibroblasts as compared to the exosomes derived from other stem cells (UC-MSC-Exo and BM-MSC-Exo), it can be inferred that the exosomes isolated from the adipose-derived stem cells (HASC-Exo) may be useful in treatment of pulmonary fibrosis.

### Example 4: Isolation of exosomes from human adipose-derived stem cells under normoxia and hypoxia conditions

In general, experiments using stem cells are conducted under normal oxygen partial pressure (oxygen 21%). However, it is known that the *in-vivo* environment where the stem cells are actually exposed in the body has a very low oxygen partial pressure (Exp Hematol., 2002; 30: 67-73).

In Example 3, it was confirmed that the exosomes isolated from the human adipose-derived stem cells (HASC-Exo) exhibit superior collagen synthesis inhibition ability in fibrosis-induced lung fibroblasts as compared to the exosomes derived from other stem cells (UC-MSC-Exo and BM-MSC-Exo) and the exosomes isolated from the adipose-derived stem cells can be usefully used for treatment of pulmonary fibrosis.

Based on this result, in order to isolate exosomes effective for treatment of pulmonary fibrosis, human adipose-derived stem cells were proliferated by subculturing until passage 7 and then cultured under a normoxia condition or a hypoxic condition to isolate exosomes (normoxia exosomes or hypoxia exosomes).

Specifically, the human adipose-derived stem cells were subcultured until passage 7 using a DMEM (Dulbecco's modified Eagle's medium, high glucose; Gibco, Cat#: 11995065) normal culture medium supplemented with 10% FBS (fetal bovine serum) and 1% penicillin/streptomycin. Cobalt chloride(II) hexahydrate (CoCl₂·6H₂O) known as a substance inducing a hypoxic condition in cultured cells was used to induce a hypoxic culture condition. After changing the medium composition by adding the cobalt chloride(II) hexahydrate to the culture medium at a concentration of 100 µM, the cells were cultured for 1 day (FIG. 5, A). Meanwhile, for a normoxic culture condition, cobalt chloride(II) hexahydrate was not added to the culture medium.

24 hours before isolating exosomes, the medium was replaced with a serum-free, antibiotic-free, phenol red-free medium (Gibco, Cat#: 31053028). After culturing for 24 hours, a cell culture supernatant was recovered. Exosomes were isolated and purified from the recovered cell culture supernatant using a multifilter system as described in Example 1.

As a result of investigating the concentration of the exosomes isolated from the human adipose-derived stem cells cultured under the normoxia condition or hypoxic condition by nanoparticle tracking analysis, the concentration of the exosomes isolated from the human adipose-derived stem cells cultured under the normoxia condition (normoxia exosomes) was 2.25×10⁹ particles/mL and the concentration of the exosomes isolated from the human adipose-derived stem cells cultured under the hypoxic condition (hypoxia exosomes) was 2.45×10⁹ particles/mL (FIG. 5, C). Also, the isolated exosomes were found to be in the form of round nanoparticles (FIG. 5, B).

### Example 5: Evaluation of therapeutic effect for pulmonary fibrosis of exosomes isolated from adipose-derived stem cells

In order to investigate the therapeutic effect for pulmonary fibrosis of the exosomes obtained in Example 4, i.e., the exosomes obtained from the adipose-derived stem cells cultured under the normoxia condition (Normoxia-Exo) and the exosomes obtained from the adipose-derived stem cells cultured under the hypoxic condition (Hypoxia-Exo), a pulmonary fibrosis animal model was established and the therapeutic effect for pulmonary fibrosis was evaluated by administering the exosomes to the established pulmonary fibrosis animal model.

Specifically, a bleomycin induced pulmonary fibrosis C57BL/6J mouse model was established by injecting a bleomycin solution, which induces pulmonary fibrosis, directly into the lungs of C57BL/6J mouse (9-week-old, male, Central Lab. Animal) by intratracheal instillation (IT). Normoxia-Exo and Hypoxia-Exo were respectively administered to the established bleomycin-induced pulmonary fibrosis C57BL/6J mouse via intravenous (IV) injection. A group administered with PBS (phosphate-buffered saline) was used as a negative control group and a group administered with human adipose-derived stem cells (HASCs) was used as a positive control group.

More specifically, the human adipose-derived stem cells (HASCs) were prepared in PBS at a concentration of 1×10⁶ cells/100 µL and each of Normoxia-Exo and Hypoxia-Exo was dispersed in PBS to a concentration of 1×10⁸ particles/100 µL (corresponding to 5×10⁹ particles/kg) for injection.

The prepared Normoxia-Exo and Hypoxia-Exo were administered to the bleomycin-induced pulmonary fibrosis C57BL/6J mouse for 3 times on days 1, 3 and 5 with 2-day intervals via intravenous injection. The PBS, as the negative control group, and the human adipose-derived stem cells (HASCs) prepared at the concentration of 1×10⁶ cells/100 µL, as the positive control group, were also administered for 3 times with 2-day intervals via intravenous injection, as the Normoxia-Exo and Hypoxia-Exo (FIG. 6, A).

On day 6, i.e., on the next day after the third administration of the PBS, HASCs, Normoxia-Exo and Hypoxia-Exo via intravenous injection, the lung tissue was extracted to investigate the therapeutic effect for pulmonary fibrosis (FIG. 6, B).

Trichrome staining was conducted on the lung tissue. The degree of pulmonary fibrosis was investigated by quantifying the blue-stained collagen area (FIGS. 7-9).

As a result, it was confirmed that the test groups to which the Normoxia-Exo and Hypoxia-Exo respectively isolated from the adipose-derived stem cells cultured under the normoxia condition and the hypoxic condition were administered showed significantly decreased stained collagen area as compared to the negative control group (PBS-administered group) or the positive control group (human adipose-derived stem cells (HASCs)-administered group), to a level similar that of the normal lung tissue.

In particular, the test group to which the exosomes isolated from the adipose-derived stem cells cultured under the hypoxic condition (Hypoxia-Exo) were administered showed remarkably decreased stained collagen area as compared to the negative control group, the positive control group and the test group administered with Normoxia-Exo, to a level nearly similar that of the normal lung tissue (FIGS. 7-9).

From these results, it was confirmed that both the exosomes obtained from the adipose-derived stem cells cultured under the normoxia condition (Normoxia-Exo) and the exosomes obtained from the adipose-derived stem cells cultured under the hypoxic condition (Hypoxia-Exo) have an effect of improving or treating pulmonary fibrosis. In particular, it can be seen that the exosomes obtained from the adipose-derived stem cells cultured under the hypoxic condition (Hypoxia-Exo) exhibit remarkably superior therapeutic effect for pulmonary fibrosis.

## Claims

1. A pharmaceutical composition for preventing or treating pulmonary fibrosis, comprising exosomes isolated from adipose-derived stem cells as an active ingredient.

2. The pharmaceutical composition according to claim 1, wherein the exosomes are isolated from adipose-derived stem cells cultured under a normoxia condition.

3. The pharmaceutical composition according to claim 1, wherein the exosomes are isolated from adipose-derived stem cells cultured under a hypoxic condition.

4. The pharmaceutical composition according to claim 3, wherein the hypoxic condition is induced by 0.5-10% oxygen or by a hypoxic cell sensitizer.

5. The pharmaceutical composition according to claim 1, wherein the exosomes comprise hepatocyte growth factor (HGF) and interleukin-10.

6. The pharmaceutical composition according to claim 1, wherein the pharmaceutical composition is for intratracheal administration or inhalation.

7. The pharmaceutical composition according to claim 1, wherein the pharmaceutical composition is an injection.

8. The pharmaceutical composition according to claim 7, wherein the injection comprises the exosomes at a concentration of 5×10⁸ to 5×10¹⁰ particles/kg.

9. A use of exosomes isolated from adipose-derived stem cells in preparation of a pharmaceutical for preventing or treating pulmonary fibrosis.

10. The use according to claim 9, wherein the exosomes are isolated from adipose-derived stem cells cultured under a normoxia condition.

11. The use according to claim 9, wherein the exosomes are isolated from adipose-derived stem cells cultured under a hypoxic condition.

12. The use according to claim 11, wherein the hypoxic condition is induced by 0.5-10% oxygen or a hypoxic cell sensitizer.

13. The use according to claim 9, wherein the exosomes comprise hepatocyte growth factor (HGF) and interleukin-10.

14. The use according to claim 9, wherein the pharmaceutical is for intratracheal administration or inhalation.

15. The use according to claim 9, wherein the pharmaceutical is an injection.

16. The use according to claim 15, wherein the injection comprises the exosomes at a concentration of 5×10⁸ to 5×10¹⁰ particles/kg.

17. A method for preventing pulmonary fibrosis, comprising administering a therapeutically effective amount of a pharmaceutical composition comprising exosomes isolated from adipose-derived stem cells as an active ingredient to a subject.

18. The method according to claim 17, wherein the exosomes are isolated from adipose-derived stem cells cultured under a normoxia condition.

19. The method according to claim 17, wherein the exosomes are isolated from adipose-derived stem cells cultured under a hypoxic condition.

20. The method according to claim 19, wherein the hypoxic condition is induced by 0.5-10% oxygen or a hypoxic cell sensitizer.

21. The method according to claim 17, wherein the exosomes comprise hepatocyte growth factor (HGF) and interleukin-10.

22. The method according to claim 17, wherein the pharmaceutical composition is for intratracheal administration or inhalation.

23. The method according to claim 17, wherein the pharmaceutical composition is an injection.

24. The method according to claim 23, wherein the injection comprises the exosomes at a concentration of 5×10⁸ to 5×10¹⁰ particles/kg.
